# EUROPEAN PATENT APPLICATION

(11) **EP 3 037 082 A1**
(43) Date of publication of application: **29.06.2016**
(21) Application number: 14004349.8
(22) Date of filing: 22.12.2014
(51) Int. Cl.: A61K 8/29, A61K 8/35, A61K 8/37, A61K 8/49, A61Q 17/00, A61Q 17/04, A61K 8/04, A61K 8/06

(54) **Sprayable cosmetic sunscreen composition**

(71) Applicant: Spirig Pharma AG, CH-4622 Egerkingen (CH)
(72) Inventor: Bohnenblust, Katharina, 4622 Egerkingen (CH); Kreutzberg, Christoph, 4622 Egerkingen (CH)
(74) Representative: Macquet, Christophe

(57) **Abstract**

The invention relates to a sprayable sun protection composition comprising, in a physiologically acceptable medium, at least one UV filter and at least one emollient. The invention is characterised in that it further comprises an emulsion stabilizing and viscosity agent, a chelating agent, a surfactant and in that it does not comprise xanthan gum or any conventional emulsifier.

## Description

This invention relates to a sprayable sun protection composition. More particularly, the invention relates to a composition formulated as a gel-spray having a Sun Protection Factor (SPF) greater than or equal to 25. The invention also relates to a method for manufacturing such a sprayable sun protection composition as well as its use, in particular for protecting the skin from UV-A and/or UV-B radiation.

Solar radiation is known to have beneficial effects but especially harmful effects in Man. Solar radiation has in particular an anti-rickets action, by assisting in the synthesis of vitamin D, improves certain dermatoses, has an antidepressant action, and allows the skin to darken. However, overexposure to solar radiation can induce an alteration of the skin.

Solar radiation comprises ultraviolet (UV) radiation which is very powerful. Among ultraviolet radiation, only UV-A and UV-B radiation reach the earth and have effects on the skin. UV-C radiation is stopped by the ozone layer.

UV-A rays, with wavelengths between 320 and 400 nm, cause the skin to darken. They are present all throughout the year and even in cloudy weather. They represent 95% of the UVs that touch the surface of the earth. Contrary to UV-B radiation, they are painless and can penetrate the skin very deeply, to the cells of the dermis. UV-A radiation in particular causes:
- photo-ageing, i.e. the modification of the orientation of the elastin and collagen fibres, causing a slackening and a loss of firmness of the skin and a premature ageing by the appearance of wrinkles;
- Sun allergies or photo-allergies (redness, itching, summer lucitis);
- Pigmentary disorders (pregnancy mask, freckles); and
- The development of skin cancers.

As such, during an exposure to natural or artificial rays, it is important to filter the UV-A radiation.

UV-B rays, with wavelengths between 280 and 320 nm are high in energy. UV-B rays are responsible for tanning but also for burns (sunburn) as well as allergic reactions and skin cancers.

In light of the above, it therefore appears essential to protect the skin from both UV-A radiation and from UV-B radiation.

Many sun protection compositions (UV-A and/or UV-B) have been proposed to date. Such compositions comprising several sun filters are in particular described in document FR 2975590 or in document FR 2983718.

There is however a need to develop sun protection compositions that are easy and fast to apply, and which are simple to use.

Such sun protection compositions must moreover comply with the regulations in terms of SPF (Sun Protection Factor.

Sun protection compositions that have a high SPF are primarily in the form of viscous creams or gels. This type of formulation makes their spreading on the skin generally long and tedious.

There remains therefore a need to have a wide range of sun protection compositions, which are easy to apply and stable.

The solution to the problem at hand has for first object a sprayable sun protection composition comprising, in a physiologically acceptable medium, at least one UV filter and at least one emollient, wherein it further comprises an emulsion stabilizing and viscosity agent, a chelating agent, and a surfactant. In a preferred embodiment, the composition does not contain xanthan gum.

Surprisingly, the Applicant has revealed that it was possible to develop sun protection compositions with high sun protection i.e. that have an SPF (Sun Protection Factor) greater than or equal to 25, that can be sprayed and that as such facilitate their application on the skin. The sprayable sun protection compositions according to the invention make it possible to apply one or several sun filters over a large surface of the skin, faster in relation to the conventional sun protection compositions. Moreover, the sun protection compositions according to the invention have good stability and do not require the use of the gelling agent xanthan gum, also marketed in particular under the name Xantural™ 75.

More preferably, the sun composition according to the invention does not require the use of any conventional emulsifier which is normally needed to obtain a stable emulsion.

The invention also has for object a method of manufacturing a composition according to the invention, wherein it comprises at least the steps of:
- preparing the oily phase by mixing and heating under agitation of a first phase comprising at least one UV filter with the film forming agent and at least one emollient;
- preparing the aqueous phase by mixing, homogenising and heating a second phase comprising at least one gelling agent, at least one emulsion stabilizing and viscosity agent, at least one chelating agent and purified water;
- emulsifying the first phase in the second phase and homogenising;
- cooling down to room temperature and adding tocopherol and ethanol;
- homogenising and recovering a composition according to the invention.

Finally, the invention has for last object the use of a composition according to the invention, for protecting the skin against UV-A and/or UV-B radiation.

The invention shall be better understood when reading the following non-restricted description.

The sun protection composition according to the invention is adapted to a topical application on the skin and therefore contains a physiologically acceptable medium, i.e. compatible with the skin. The compositions according to the invention comprise a physiologically acceptable medium or at least one pharmaceutically acceptable excipient, chosen according to the pharmaceutical or dermatological form desired.

The sun protection composition according to the invention can have for example the form of an oily solution, of a dispersion of the lotion type that is possibly two-phased, of an emulsion obtained by the dispersion of an oily phase in an aqueous phase (O/W) or inversely (W/O), or a triple emulsion (W/O/W or C/W/O).

More preferably, the sun protection composition is an emulsion obtained by the dispersion of an oily phase in an aqueous phase (O/W).

The sun protection composition according to the invention can be sprayed. As such, the final product can have the form of an aerosol, an atomizer, a mister, a gel-spray, a nebulizer, a spray or a vaporizer. The sun protection composition according to the invention is fluid, i.e. it is in liquid form which allows it to be sprayed. The composition is moreover in a form that is adapted to topical application on the skin.

More preferably, the sun protection composition according to the invention is a gel-spray.

The sun protection composition according to the invention comprises at least one UV filter.

By way of a non-restricted example of a UV filter that can be used, the following can be mentioned:
- derivatives of 1,3,5-triazine-2,4,6-triamine, such as Diethylamino Hydroxybenzoyl Hexyl Benzoate (Uvinul™ A+), Ethyl Hexyl Triazone (Uvinul™ T 150), Bis-ethyl Hexyloxyphenol Methoxyphenyl Triazine (Tinosorb™ S), Octocrylene (Eusolex® OCR), Dimethicodiethylbenzalmalonate (Parsol® SLX), Di-ethylhexyl-butamido Triazone (Uvasorb® HEB);
- esters of cinnamic acid, such as isoamyl-p-methoxycinnamate or 2-ethylhexyl-p-methoxycinnamate (INCI; Ethyl Hexyl Methoxycinnamate), in particular marketed under the name Eusolex® 9020) ;
- derivatives of phenylbenzimidazole, such as Phenylbenzimidazole Sulfonix Acid, (Eusolex® 232);
- derivatives of benzophenone, such as Benzophenone-3 (Tinosorb™ B3), Benzophenone-4 (Uvinul™ MS 40) or Benzophenone-5; and
- derivatives of p-aminobenzoic acid, such as the ethoxylated derivative PEG 25 - PABA (Uvinul™ P25).

More preferably, the composition according to the invention comprises at least one UV filter chosen from among Bis-Ethylhexyloxyphenol Methoxyphenyl Triazine (BEMT) (Tinosorb™ S), ethylhexyl Methoxycinnamate or diethylamino Hydroxybenzoyl Hexyl Benzoate (Uvinul™ A+).

Advantageously, the composition according to the invention comprises at least two UV filters.

More preferably, the composition according to the invention comprises at least two UV filters chosen from among Bis-Ethylhexyloxyphenol Methoxyphenyl Triazine (BEMT) (Tinosorb™ S), ethylhexyl Methoxycinnamate and/or Diethylamino Hydroxybenzoyl Hexyl Benzoate (Uvinul™ A+).

More advantageously, the composition according to the invention comprises at least three UV filters.

More preferably, the three UV filters are Bis-Ethylhexyloxyphenol Methoxyphenyl Triazine (BEMT) (Tinosorb™ S), ethylhexyl Methoxycinnamate and diethylamino Hydroxybenzoyl Hexyl Benzoate (Uvinul™ A+).

According to an advantageous embodiment of the invention the composition comprises one or several UV filters such as hereinabove, at concentrations that allow for the obtaining of a high SPF (Sun Protection Factor), i.e. of at least 25, preferably of at least 30. More preferably, the composition having a SPF of at least 25 (and presenting an UVA-PF of 1/3 of the SPF) comprises a mixture of UV filters:
- Bis-Ethylhexyloxyphenol Methoxyphenyl Triazine (BEMT) (Tinosorb™ S),
- Ethylhexyl Methoxycinnamate and
- Diethylamino Hydroxybenzoyl Hexyl Benzoate (Uvinul™ A+).

Even more preferably, the composition having a SPF of at least 25 comprises a mixture of the following UV filters:
- between 2.0% and 5.0% of Bis-Ethylhexyloxyphenol Methoxyphenyl Triazine (BEMT) (Tinosorb™ S) ;
- between 7.0% and 10% of Ethylhexyl Methoxycinnamate; and
- between 3.5% and 4.5% of Diethylamino Hydroxybenzoyl Hexyl Benzoate (Uvinul™ A+).

For example, a composition containing the following UV filters:
- 2.5% of Bis-Ethylhexyloxyphenol Methoxyphenyl Triazine (BEMT) (Tinosorb™ S);
- 10% of Ethylhexyl Methoxycinnamate; and
- 4.0% of Diethylamino Hydroxybenzoyl Hexyl Benzoate (Uvinul™ A+)
has a calculated SPF of 31.7 and a calculated UVA-PF of 8.5.

In the same way, a composition containing the following UV filters:
- 5% of Bis-Ethylhexyloxyphenol Methoxyphenyl Triazine (BEMT) (Tinosorb™ S);
- 7% of Ethylhexyl Methoxycinnamate; and
- 4.0% of Diethylamino Hydroxybenzoyl Hexyl Benzoate (Uvinul™ A+)
has a SPF of 32.6 and an UVA-PF of 11.4.

In the same way, a composition containing the following UV filters:
- 2% of Bis-Ethylhexyloxyphenol Methoxyphenyl Triazine (BEMT) (Tinosorb™ S);
- 8% of Ethylhexyl Methoxycinnamate; and
- 3.5% of Diethylamino Hydroxybenzoyl Hexyl Benzoate (Uvinul™ A+)
has a SPF of 25.7 and an UVA-PF of 8.9.

The composition according to the invention further comprises at least one emollient. The emollient makes it possible in particular to solubilise the UV filters and to prevent the recristallisation thereof.

By way of a non-restricted example of an emollient that can be used, the following can be mentioned:
- benzoate esters such as Dipropylene Glycol Dibenzoate (Finsolv® PG-22), Ethylhexyl Benzoate (Finsolv® EB) or C12-15 Alkyl Benzoate;
- Dicaprylyl Carbonate (Cetiol® CC) and
- Dibutyl Adipate.

More preferably, the emollient is chosen from among C12-15 Alkyl Benzoate or Dibutyl Adipate.

Advantageously, the composition according to the invention comprises at least two emollients.

More preferably, the two emollients are C12-15 Alkyl Benzoate and Dibutyl Adipate.

Surprisingly, the Applicant was able to reveal that, in order to obtain a sprayable sun protection composition according to the invention which has the form of a stable oil in water (O/W) emulsion, the droplets of oil have to be stabilised by the association of at least one emulsion stabilizing and viscosity agent, a chelating agent, a surfactant.

As such, the composition according to the invention necessarily comprises an emulsion stabilizing and viscosity controlling agent. An emulsion stabilizing and viscosity controlling agent is an agent creating a kind of network where droplets are fixed. Preferably, the emulsion stabilizing agent is a polymer. More preferably, this polymer does not increase the viscosity. By way of a non-restricted example of emulsion stabilizing and viscosity controlling agent that can be used, the following can be mentioned:
- acrylate and acrylamide polymers such as Acrylates/C10-30 Alkyl Acrylate Crosspolymer (Pemulen™ TR2).

The composition according to the invention furthermore comprises a chelating agent.

By way of a non-restricted example of a chelating agent that can be used, alpha-cyclodextrin (Cavamax™ W6 Food) or trisodium Ethylenediamine Disuccinate (Natrlquest™ E30) can be mentioned. These two chelating agents are preferably used because they enable droplet stabilisation.

More preferably, the chelating agent is alpha-cyclodextrin (Cavamax™ W6 Food).The composition according to the invention further comprises a surfactant which makes it possible to reduce the surface tension and to favour an even distribution of the product when it is used.

By way of a non-restricted example of surfactant that can be used, Lauryl Glucoside (Plantacare™ 1200 UP) can be mentioned.

Surprisingly, the Applicant was able to show that the absence of xanthan gum (Xantural™ 75) in the preparation of the composition according to the invention makes it possible to develop a product having an optimum viscosity to be sprayed for example in a formulation as a gel-spray.

As such, the composition according to the invention does not contain xanthan gum such as for example Xantural™ 75.

Advantageously, the composition according to the invention does not contain any emulsifying agent, i.e. no compound that favours the formation of intimate mixtures between immiscible liquids such as oil and water. Such conventional emulsifying agents are for example chosen from among Lecithin, Cetyl Phosphate, Eumulgin VL 75 (Lauryl Glucoside (and) Polyglyceryl-2 Dipolyhydroxystearate (and) Glycerin), Montanov™ 68 (Cetearyl Alcohol and Cetearyl Glucoside), Polyglycerin-Emulsifiers.

Advantageously, the composition according to the invention further comprises one or several components, taken individually or as a mixture, such as:
- film-forming agent, such as acetyl tributyl citrate, Antaron™ polymers such as the VP/Hexadecene copolymer (Antaron™ (Ganex) V-216 Polymer), the VP/Eicosene copolymer (Antaron™ (Ganex) V-220/220F polymer and Triacontanyl PVP (Antaron™ (Ganex) WP-660 polymer); waxes such as in particular Cera Alba, Cera candelilla or Cera carnauba; cellulose and derivatives thereof (acetates, nitrates, etc.); and chitosan and derivatives thereof;
- pH regulators such as Triethanolamine (Trolamine™ EP) and Sodium Hydroxide solution;
- skin-conditioning agents such as Dimethicone 5 CS;
- antioxidants such as dl-alpha-Tocopherol;
- co-solvents such as ethanol; and
- purified water.

According to a preferred embodiment of the invention, the sprayable sun protection composition according to the invention does not contain xanthan gum, but comprises at least:
- one UV filter which is chosen from among Bis-Ethylhexyloxyphenol Methoxyphenyl Triazine (BEMT) (Tinosorb™ S), ethylhexyl Methoxycinnamate or diethylamino Hydroxybenzoyl Hexyl Benzoate (Uvinul™ A+), taken individually or as a mixture;
- one emollient which is C12-15 Alkyl Benzoate and/or Dibutyl Adipate;
- one emulsion stabilizing and viscosity agent which is Acrylates/C10-30 Alkyl Acrylate Crosspolymer (Pemulen™ TR2);
- one chelating agent which is alpha-cyclodextrin (Cavamax™ W6 Food); and
- one surfactant which is Lauryl Glucoside (Plantacare™ 1200 UP).

More preferably, the composition further comprises:
- purified water;
- trisodium Ethylenediamine Disuccinate (Natrlquest™ E30);
- Triethanolamine (Trolamine™ EP);
- VP/Eicosene Copolymer (Antaron™ V-220 F);
- Dimethicone;
- dl-alpha-Tocopherol; and
- ethanol.

Even more preferably, the composition according to the invention comprises the following constituents in the following proportions, expressed as a percentage in weight of the total weight of the composition:
- 40% to 70% purified water;
- 0.01% to 0.1% Acrylates/C10-30 Alkyl Acrylate Crosspolymer (Pemulen™ TR2);
- 0.01% to 0.5% trisodium Ethylenediamine Disuccinate (Natrlquest™ E30);
- 0.1% to 5.0% alpha-cyclodextrin (Cavamax™ w6 Food)
- 0.01% tc 0.5% Triethanolamine (Trolamine™ EP)
- 0.5% to 5.0% C12-15 Alkyl Benzoate;
- 0.5% to 5.0% Dibutyl Adipate;
- 0.05% to 5.0% VP/Eicosene Copolymer (Antaron™ V-220 F)
- 2.0% to 5.0% Bis-Ethylhexyloxyphenol Methoxyphenyl Triazine (BEMT) (Tinosorb™ S);
- 7.0 to 10% ethylhexyl Methoxycinnamate;
- 3.5% to 5 % Diethylamino Hydroxybenzoyl Hexyl Benzoate (Uvinul™ A+);
- 0.05% to 6.0% Dimethicone 5 CS;
- 0.1% to 3.0% dl-alpha-Tocopherol;
- 2.0% to 20% ethanol 96% EP;
- 0.005% to 2.0% Lauryl Glucoside (Plantacare™ 1200 UP)

Particularly advantageously, the composition according to the invention comprises the following constituents in the following proportions, expressed as a percentage in weight of the total weight of the composition:
- 55% to 65% purified water:
- 0.01% to 0.1% Acrylates/C10-30 Alkyl Acrylate Crosspolymer (Pemulen™ TR2);
- 0.05% to 0.2% trisodium Ethylenediamine Disuccinate (Natrlquest™ E30) :
- 0.5% to 2.0% alpha-cyclodextrin (Cavamax™ W6 Focd);
- 0.05 to 0.08% Triethanolamine (Trolamine™ EP);
- 1.5% to 3.0% C12-15 Alkyl Benzoate;
- 1.0% to 3.0% Dibutyl Adipate;
- 0.1% to 3.0% VP/Eicosene Copolymer (Antaron™ V-220 F);
- 2.0% to 5.0% Bis-Ethylhexyloxyphenol Methoxyphenyl Triazine (BEMT) (Tinosorb™ S);
- 7.0% tc 10% ethylhexyl Methoxycinnamate;
- 3.5% to 4.5% Diethylamino Hydroxybenzoyl Hexyl Benzoate (Uvinul™ A+) ;
- 0.1% to 5.0% Dimethicone 5 CS;
- 0.1% to 2.0% dl-alpha-Tocopherol;
- 6% to 13% ethanol 96% EP; and
- C.01% to 0.15% Lauryl Glucoside (Plantacare™ 1200 UP).

The invention also relates to a method of manufacturing a composition according to the invention, wherein it comprises at least the steps of:
- preparing the oily phase by mixing and heating under agitation of a first phase comprising at least one UV filter with the film forming agent and at least one emollient;
- preparing the aqueous phase by mixing, homogenising and heating a second phase comprising at least one gelling agent, at least one emulsion stabilizing and viscosity agent, at least one chelating agent and purified water;
- emulsifying the first phase in the second phase and homogenising;
- cooling down to room temperature and adding tocopherol and ethanol; and
- homogenising and recovering a composition according to the invention.

More preferably, the first phase further comprises VP/Eicosene Copolymer (Antaron™ V-220 F) and Dimethicone, the second phase further comprises trisodium Ethylenediamine Disuccinate (Natrlguest™ E30), and the third phase further comprises dl-alpha-Tocopherol- and ethanol.

Even more preferably, prior to the step of emulsifying of the first phase in the second phase and homogenising, triethanolamine is added to the second phase.

Finally, the invention relates to the use of a composition according to the invention, for protecting the skin against UV-A and/or UV-B radiation.

More preferably, the compositions are particularly effective in protecting human skin against the harmful effects of UV-A and/or UV-B radiation which include but are not limited to photo-ageing, burns, sun allergies or photo-allergies, pigmentary disorders and the development of skin cancers.

As such, this invention also relates to a method for protecting human skin against the harmful effects of UV-A and/or UV-B radiation, by application of a composition according to the invention.

This invention shall now be illustrated by means of the following examples. In these examples, the quantities are expressed as a percentage by weight.

### EXAMPLES

### Example 1: sprayable sun protection compositions:

The Applicant manufactured and analysed five different sun protection compositions numbered 26, 27, 63, 66 and 122.

Table 1 hereinbelow provides the qualitative and quantitative content of the compositions tested:

**Table 1:**

| | | **Compositions** | | | | |
|---|---|---|---|---|---|---|
| | | **26** | **27** | **63** | **66** | **122** |
| **Phrases** | **Constituents** | **%** | **%** | **%** | **%** | **%** |
| **A** | Purified water (qsp) | from 55.000% to 65.000% | | | | |
| | Pemulen TR2 (Acrylates/C10-30 Alkyl Acrylate Crosspolymer) | from 0.010% to 0.150% | | | | |
| | Xantural™ 75 (Xanthan Gum) | from 0.100% to 0.300% | from 0.100% to 0.300% | - | from 0.100% to 0.300% | - |
| | Natrlquest™ E30 (trisodium Ethylenediamine Disuccinate) | from 0.050% to 0.200% | | | | |
| | Cavamax™ W6 Food (alpha-cyclodextrin) | from 0.500% to 2.000% | | | | |
| | Trolamine™ EP (Triethanolamine) | from 0.050% to 1.000% | | | | |
| | Plantacare™ 1200 UP (Lauryl Glucoside) | - | - | from 0.010% to 0.150% | | |
| B | C12-15 Alkyl Benzoate | from 1.500% to 3.500% | - | from 1.500% to 3.500% | - | from 1.500% to 3.500% |
| | Dibutyl Adipate | from 1.000% to 3.000% | - | from 1.000% to 3.000% | - | from 1.000% to 3.000% |
| | Antaron™ V-220 F (VP/Eicosene Copolymer) | from 0.100% to 3.000% | | | | |
| | Tinosorb™ S (Bis-Ethylhexyloxyphen ol Methoxyphenyl Triazine (BEMT) | from 2.000% to 5.000% | | | | |
| | Ethylhexyl Methoxycinnamate | from 7.000% to 10.000% | | | | |
| | Uvinul™ A+ (Diethylamino Hydroxybenzoyl Hexyl Benzoate) | from 3.500% to 5.000% | | | | |
| | Dimethicone 5 CS | from 0.100% to 5.000% | | | | |
| c | dl-alpha-Tocopherol# | from 0.100% to 2.000% | | | | |
| | Ethanol 96% EP | from 6.000% to 13.000% | | | | |

### Example 2: Method for manufacturing sun protection compositions according to example 1:

The compositions 26, 27, 63 and 122 were prepared according to the following steps:
1. weighing of the various constituents of phase B in a glass beaker;
2. melting of the various constituents of phase B under agitation using a magnetic stirrer hot plate;
3. weighing of the various constituents of phase A, except for Trolamine™ EP, in a glass beaker;
4. mixing and homogenising of the various constituents of phase A;
5. weighing of Trolamine™ EP in the phase A;
6. mixing and homogenising of phase A;
7. heating of phase A to 80°C;
8. emulsifying of phase B in phase A by homogenising;
9. cooling the gel to room temperature; and
10. adding phase C and homogenising of the whole.

### Example 3: Pre-selection of the sun protection compositions according to the example 1 to be analysed:

Among the four compositions manufactured the compositions 27 and 66, which do not contain lipids, were not retained for the remainder of the tests. Indeed, the absence of lipids in these compositions causes the UV filters to re-crystallise.

Moreover, the excessive viscosity of the composition 66, which comprises both Plantacare™ 1200 UP and Xantural™ 75, does not allow it to be formulated in the form of a spray or a gel-spray.

As such, only the compositions 26 (used as reference composition with Xanthan gum), 63 and 122 were evaluated both under the microscope and via a temperature test.

This example demonstrates that any composition does not fulfil the features to be sprayable according to invention. The addition of Xanthan gum, does not provide a sprayable composition. Indeed, the composition 26 with Xanthan gum is not sprayable.

### Example 4: Galenic evaluation of the composition 26:

The quality and the stability of the composition 26 were evaluated under the microscope and by a temperature test.

The results of the evaluation under the microscope of the composition 26 (Axioskop, Zeiss) are provided in table 2 hereinbelow:

**Table 2:**

| **Time (weeks)** | **Evaluation with polarizer** | **Evaluation on brightfield** |
|---|---|---|
| T0 (After manufacture) | Absence of crystals | Homogeneous composition No presence of droplets |
| T+4 weeks at room temperature (RT) | Absence of crystals | Homogeneous composition Droplets with a size of less than 5µm |
| T+8 weeks at RT | Absence of crystals | Homogeneous composition Droplets with a size of less than 5µm |
| T+8 weeks at +4°C | Absence of crystals | Homogeneous composition Droplets with a size of less than 5µm |
| T+12 weeks at RT | Absence of crystals | Homogeneous composition Droplets with a size of less than 10µm |
| T+12 weeks at +4°C | Absence of crystals | Homogeneous composition Droplets with a size of less than 10µm |
| T+26 weeks at RT | Absence of crystals | Homogeneous composition Droplets with a size of less than 10µm |
| T+26 weeks at +4°C | Absence of crystals | Homogeneous composition Droplets with a size of less than 10µm |

The results of the temperature tests of the composition 26 are provided in table 3 hereinbelow. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 3:**

| **Time (weeks)** | **Macroscopic and sensory properties of the composition** |
|---|---|
| T0 (After manufacture) | Smooth, yellow, shiny composition with characteristic odour, having minimum fluidity |
| T+2 weeks at room temperature (RT) and 60 °C alternating (Stresstest) | Identical to T0 |
| T+4 weeks at +4°C | Identical to T0 |
| T+4 weeks at RT | Increased fluidity or otherwise identical to T0 |
| T+4 weeks at +30°C | Identical to T0 |
| T+4 weeks at +40°C | Increased fluidity or otherwise identical to T0 |
| T+8 weeks at +4°C | Identical to T0 |
| T+8 weeks at RT | Increased fluidity or otherwise identical to T0 |
| T+8 weeks at +3C°C | Identical to TC |
| T+8 weeks at +40°C | Increased fluidity or otherwise identical to T0 |
| T+12 weeks at +4°C | Identical to T0 |
| T+12 weeks at RT | Increased fluidity or otherwise identical to T0 |
| T+12 weeks at +30°C | Identical to T0 |
| T+12 weeks at +40°C | Increased fluidity or otherwise identical to T0 |
| T+26 weeks at +4°C | Identical to T0 |
| T+26 weeks at RT | Identical to T0 |
| T+26 weeks at +30°C | Identical to T0 |
| T+26 weeks at +40°C | Increased fluidity or otherwise identical to T0 |

The results shown in tables 2 and 3 hereinabove, show that the composition 26 has qualities that allow it to be formulated as a gel-cream formulation. However, the results of the galenic evaluation provided in table 2 also show that the composition 26 does not have sufficient stability.

Also, in order to increase the stability, the Applicant developed the composition 66, which comprises, in addition to the xanthan gum, Lauryl Glucoside.

However, as indicated hereinabove, the composition 66 obtained, which contains both Plantacare™ 1200 UP and Xantural™ 75, is too viscous, which does not allow it to be formulated in the form of a spray or a gel-spray.

### Example 5: Galenic evaluation of the composition 63:

Among the various compositions developed by the Applicant, the composition 63, which is substantially identical to the composition 66 (not retained as it is too viscous) detailed hereinabove, drew the attention of the Applicant. Indeed, it is following the omission of an ingredient during its preparation that the composition 63 was developed. As detailed in table 1 hereinabove, the composition 63 is distinguished from the composition 66 in that it does not contain xanthan gum (Xantural™ 75).

As with the composition 26, the quality and the stability of the composition 63 were evaluated under the microscope and by a temperature test.

The results of this evaluation under the microscope (Axioskop, Zeiss) are provided in table 4 hereinbelow:

**Table 4:**

| **Time (weeks)** | **Evaluation with polarizer** | **Evaluation on brightfield** |
|---|---|---|
| T0 (After manufacture) | Absence of crystals | Homogeneous composition, structure of the gel type No presence of droplets |
| T+4 weeks at room temperature (RT) | Identical to T0 | Identical to T0 |
| T+8 weeks at RT | Identical to T0 | Identical to T0 |
| T+8 weeks at +4°C | Identical to T0 | Identical to T0 |
| T+12 weeks at RT | Identical to T0 | Identical to T0 |
| T+12 weeks at +4°C | Identical to T0 | Identical to T0 |
| T+26 weeks at RT | Identical to T0 | Identical to T0 |
| T+26 weeks at +4°C | Identical to T0 | Identical to T0 |

The results of the temperature tests of the composition 63 are provided in table 5 hereinbelow. During the temperature tests, sensory and macroscopic parameters are verified, such as in particular the colour, the change in colour, the odour, the shininess, the superficial properties, the consistency, the oily and/or aqueous deposit, the granularity, formation of a protuberance.

**Table 5:**

| **Time (weeks)** | **Macroscopic and sensory properties of the composition** |
|---|---|
| T0 (After production) | Smooth, yellow, shiny composition, with an odour of ethanol, having good fluidity |
| T+2 weeks at room temperature (RT) and 60 °C alternating (stress test) | Identical to T0 |
| T+4 weeks at +4°C | Identical to T0 |
| T+4 weeks at RT | Identical to T0 |
| T+4 weeks at +30°C | Identical to T0 |
| T+4 weeks at +40°C | Start of heterogeneity, separation of the oily phase |
| T+8 weeks at +4°C | Identical to T0 |
| T+8 weeks at RT | Identical to T0 |
| T+8 weeks at +30°C | Identical to T0 |
| T+8 weeks at +40°C | Identical to T0 (no separation of the oily phase or heterogeneity visible) |
| T+12 weeks at +4°C | Identical to T0 |
| T+12 weeks at RT | Identical to T0 |
| T+12 weeks at +30°C | Identical to T0 |
| T+12 weeks at +40°C | Slight separation of the oily phase on the surface |
| T+26 weeks at +4°C | Identical to T0 |
| T+26 weeks at RT | Identical to T0 |
| T+26 weeks at +30°C | Identical to T0 |
| T+26 weeks at +40°C | Slight separation of the oily phase on the surface |

The results shown in tables 4 and 5 hereinabove, show that the composition 63 has both good microscopic stability over time (absence of crystals and droplets even after 26 weeks) and good resistance at high temperatures. This composition 63 also has physical-chemical properties that make it particularly adapted for a formulation as a gel-spray.

Surprisingly, the Applicant was able to reveal that the fortuitous absence of xanthan gum in the preparation of the composition 63, made it possible to develop a composition that has a viscosity that is acceptable for a formulation as a gel-spray.

The composition 63 obtained is stable and does not require any emulsifying agent.

When the composition 63 is compared with the composition 26 which does not contain Lauryl Glucoside (Plantacare™ 1200 UP), it can be deduced that it is possible to substitute xanthan gum (Xantural™ 75) with Lauryl Glucoside (Plantacare™.1200 UP) in order to obtain a composition as a gel-spray.

The Applicant was therefore able to develop a composition formulated as a gel-spray that has a high protection factor (PF) or "SPF" (for Sun Protection Factor), i.e. greater than or equal to 25.

### Example 6: Galenic evaluation of the composition 122: Starting with the composition 63, the Applicant also optimised the quantity of ingredients in order to develop a composition formulated as a gel-spray that has a protection factor (PF) of at least 30.

Among the various compositions developed by the Applicant, the composition 122, which is substantially identical to the composition 63 detailed hereinabove, met the highest requirements of the Applicant. As detailed in table 1 hereinabove, the composition 122 is distinguished from the composition 63 in that it comprises different concentrations of triethanolamine, of UV filters Tinosorb™ S and Uvinul™ A+.

As for the compositions 26 and 63, the quality and the stability of the composition 122 were evaluated under the microscope and by a temperature test.

The results of this evaluation under the microscope (Axioskop, Zeiss) are provided in table 6 hereinbelow:

**Table 6:**

| Time (weeks) | With polarizer | On brightfield |
|---|---|---|
| T0 | Absence of | Homogeneous composition. |

| | crystals | Nc presence of droplets |
|---|---|---|
| T+4 weeks at room temperature | Identical to T0 | Identical to T0 |
| T+8 weeks at room temperature | Identical to T0 | Identical to T0 |
| T+8 weeks at +4°C | Identical to T0 | Identical to T0 |
| T+12 weeks at room temperature | Identical to T0 | Identical to T0 |
| T+12 weeks at +4°C | Identical to T0 | Identical to T0 |
| T+2E weeks at room temperature | Identical to T0 | Identical to T0 |
| T+26 weeks at +4°C | Identical to T0 | Identical to T0 |

The results of the temperature tests of the composition 122 are provided in table 7 hereinbelow. During the temperature tests, sensory and macroscopic parameters are verified, such as in particular the colour, the change in colour, the odour, the shininess, the superficial properties, the consistency, the oily and/or aqueous deposit, the granularity, formation of a protuberance.

**Table 7:**

| Time (weeks) | Macroscopic and sensory properties of the composition |
|---|---|
| T0 (After production) | Smooth, yellow, shiny composition with an odour of ethanol, having good fluidity and air inclusions |
| T+4 weeks at +4 °C | Identical to T0 |
| T+4 weeks at room temperature (RT) | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | Identical to T0, but difficult to spray |
| T+8 weeks at +30°C | Slight separation of the oily phase on the surface and difficult to spray |
| T+8 weeks at +40°C | Slight separation of the oily phase on the surface and difficult to spray |
| T+12 weeks at +4°C | Identical to T0 |
| T+12 weeks at RT | Identical to T0, but difficult to spray |
| T+12 weeks at +30°C | Slight separation of the oily phase on the surface and difficult to spray |
| T+12 weeks at +40°C | Slight separation of the oily phase on the surface and difficult to spray |
| T+26 weeks at +4°C | Identical to T0 |
| T+26 weeks at RT | Identical to T0, but difficult to spray |
| T+26 weeks at +30°C | Slight separation of the oily phase on the surface and difficult to spray |
| T+26 weeks at +40°C | Slight separation of the oily phase on the surface and difficult to spray |

The results shown in tables 6 and 7 hereinabove, show that the composition 122 has, as composition 63, both good microscopic stability over time (absence of crystals and droplets even after 26 weeks) and good resistance to high temperatures. This composition 122 also has physical-chemical properties the make it particularly adapted for a formulation as a gel-spray.

The Applicant was as such able to develop a composition formulated as a gel-spray having a high SPF), i.e. of at least 30.

## Claims

1. Sprayable sun protection composition comprising, in a physiologically acceptable medium, at least one UV filter and at least one emollient,
wherein it further comprises an emulsion stabilizing and viscosity agent, a chelating agent , a surfactant and in that it does not contain xanthan gum.

2. Composition according to claim 1, wherein:
- the UV filter is chosen from among Bis-Ethylhexyloxyphenol Methoxyphenyl Triazine (BEMT) (Tinosorb™ S), ethylhexyl Methoxycinnamate or diethylamino Hydroxybenzoyl Hexyl Benzoate (Uvinul™ A+), taken individually or as a mixture;
- the emollient is C12-15 Alkyl Benzoate and/or Dibutyl Adipate;
- the emulsion stabilising and viscosity agent is Acrylates/C10-30 Alkyl Acrylate Crosspolymer (Pemulen™ TR2);
- the chelating agent is alpha-cyclodextrin (Cavamax™ W6 Food); and
- the surfactant is Lauryl Glucoside (Plantacare™ 1200 UP).

3. Composition according to one of the preceding claims, wherein it further comprises:
- purified water;
- Triethanolamine (Trolamine™ EP);
- VP/Eicosene Copolymer (Antaron™ V-220 F);
- Dimethicone (Dimethicone 5 CS);
- dl-alpha-Tocopherol; and
- ethanol.

4. Composition according to one of the preceding claims, wherein it comprises the following constituents in the following proportions, expressed as a percentage in weight of the total weight of the composition:
- 40% to 70% purified water:
- 0.01% to 0.5% Acrylates/C10-30 Alkyl Acrylate Crosspolymer (Pemulen™ TR2);
- 0.01% to 0.5% trisodium Ethylenediamine Disuccinate (Natrlquest™ E30);
- 0.1% to 5% alpha-cyclodextrin (Cavamax™ W6 Food);
- 0.01% to 0.5% Triethanolamine (Trolamine™ EP) ;
- 0.5% to 5% C12-15 Alkyl Benzoate;
- 0.5% to 5% Dibutyl Adipate;
- 0.05% to 5% VP/Eicosene Copolymer (Antaron™ V-220 F);
- 0.5% to 5% Bis-Ethylhexyloxyphenol Methoxyphenyl Triazine (BEMT) (Tinosorb™ S);
- 2.0% to 20% ethylhexyl Methoxycinnamate;
- 1.0% to 8.0% Diethylamino Hydroxybenzoyl Hexyl Benzoate (Uvinul™ A+) ;
- 0.05% to 6% Dimethicone 5 CS;
- 0.1% to 3% dl-alpha-Tocopherol;
- 2.0% to 20% ethanol 96% EP; and
- 0.005% to 2% Lauryl Glucoside (Plantacare™ 1200 UP)

5. Composition according to claim 4, wherein it comprises the following constituents in the following proportions, expressed as a percentage in weight of the total weight of the composition:
- 55% to 65% purified water:
- 0.01% to 0.1% Acrylates/C10-30 Alkyl Acrylate Crosspolymer (Pemulen™ TR2);
- 0.05% to 0.2% trisodium Ethylenediamine Disuccinate (Natrlquest™ E30);
- 0.5% to 2.0% alpha-cyclodextrin (Cavamax™ W6 Food);
- 0.05 to 0.08% Triethanolamine (Trolamine™ EP);
- 1.5% to 3.0% C12-15 Alkyl Benzoate;
- 1.0% to 3.0% Dibutyl Adipate;
- 0.1% to 3.0% VP/Eicosene Copolymer (Antaron™ V-220 F);
- 2.0% to 5.0% Bis-Ethylhexyloxyphenol Methoxyphenyl Triazine (BEMT) (Tinosorb™ S);
- 7.0% to 10% ethylhexyl Methoxycinnamate;
- 3.5% to 4.5% Diethylamino Hydroxybenzoyl Hexyl Benzoate (Uvinul™ A+) ;
- 0.1% to 5.0% Dimethicone 5 CS;
- 0.1% to 2.0% dl-alpha-Tocopherol;
- 6% to 13% ethanol 96% EP; and
- 0.01% to 0.15% Lauryl Glucoside (Plantacare™ 1200 UP).

6. Composition according to one of the preceding claims, wherein it does not contain any emulsifier.

7. Composition according to one of the preceding claims, wherein it has the form of an aerosol, an atomizer, a mister, a gel-spray, a nebulizer, a spray or a vaporizer.

8. Composition according to claim 7, wherein it has the form of a gel-spray.

9. Composition according to one of the preceding claims, wherein it comprises one or several UV filters, at concentrations that make it possible to obtain a protection factor of at least 25.

10. Method of manufacturing a composition according to any of claims 1 to 9, wherein it comprises at least the steps of:
- preparing the oily phase by mixing and heating under agitation of a first phase comprising at least one UV filter with the film forming agent and at least one emollient;
- preparing the aqueous phase by mixing, homogenising and heating a second phase comprising at least one gelling agent, at least one emulsion stabilizing and viscosity agent, at least one chelating agent and purified water;
- emulsifying the first phase in the second phase and homogenising;
- cooling down to room temperature and adding tocopherol and ethanol;
- homogenising and recovering a composition according to one of claims 1 to 9.

11. Use of a composition according to one of the preceding claims, for protecting the skin against UV-A and/or UV-B radiation.
